Europäisches Patentamt

European Patent Office

Office européen des brevets

(19)

(11) Veröffentlichungsnummer: **0 229 252 B1**

(12) **EUROPÄISCHE PATENTSCHRIFT**

(45) Veröffentlichungstag der Patentschrift: **01.07.92**

(21) Anmeldenummer: **86115554.7**

(22) Anmeldetag: **10.11.86**

(51) Int. Cl.5: **C12P 7/44**, C12P 7/62,
C07C 57/13, C07C 59/42,
//(C12P7/44,C12R1:73),
(C12N1/16,C12R1:73)

(54) **Verfahren zur Herstellung von Dicarbonsäuren.**

(30) Priorität: **18.11.85 DE 3540834**

(43) Veröffentlichungstag der Anmeldung:
**22.07.87 Patentblatt 87/30**

(45) Bekanntmachung des Hinweises auf die
Patenterteilung:
**01.07.92 Patentblatt 92/27**

(84) Benannte Vertragsstaaten:
**AT BE CH DE FR GB IT LI NL SE**

(56) Entgegenhaltungen:
**US-A- 3 843 466**
**US-A- 4 474 882**

**CHEMICAL ABSTRACTS, Band 100, 1984, Seite 460, Zusammenfassung Nr. 50061z, Columbus, Ohio, US; & JP-A-58 165 793 (BIO
RESEARCH CENTER CO., LTD) 30-09-1983**

**CHEMICAL ABSTRACTS, Band 100, 1984, Seite 460, Zusammenfassung Nr. 50063b, Columbus, Ohio, US; & JP-A-58 165 794 (BIO
RESEARCH CENTER CO., LTD) 30-09-1983**

(73) Patentinhaber: **Henkel Kommanditgesellschaft
auf Aktien
Postfach 1100 Henkelstrasse 67
W-4000 Düsseldorf-Holthausen(DE)**

(72) Erfinder: **Schindler, Joachim, Dr.
Am Eichelkamp 158
W-4010 Hilden(DE)**
Erfinder: **Viehweg, Holger, Dr.
Weissenstein 83
W-4018 Langenfeld(DE)**
Erfinder: **Schmid, Rolf, Dr.
Am Nettchesfeld 30
W-4000 Düsseldorf 12(DE)**
Erfinder: **Weiss, Albrecht, Dr.
Forellenweg 37
W-4018 Langenfeld(DE)**
Erfinder: **Ott, Karl-Heinz, Dr.
Trillser Graben 6
W-4006 Erkrath(DE)**
Erfinder: **Eierdanz, Horst, Dr.
Verbindungsstrasse 5
W-4010 Hilden(DE)**

EP 0 229 252 B1

## Beschreibung

Die Erfindung betrifft ein fermentatives Verfahren zur Herstellung von Dicarbonsäuren aus längerkettigen Alkanen, primären Alkoholen, Carbonsäuren oder Carbonsäureestern. Die Verfahrensprodukte weisen einen höheren Grad an CC-Doppelbindungen auf als die Ausgangssubstanzen.

Dicarbonsäuren mit mehr als 10 C-Atomen sind nach Methoden der präparativen organischen Chemie im technischen Maßstab schwer herstellbar. Dies gilt insbesondere für Dicarbonsäuren, die außer den beiden endständigen Carboxylgruppen noch weitere funktionelle Gruppen wie Doppelbindungen oder Hydroxylgruppen aufweisen.

Man hat daher versucht, aus gut zugänglichen Rohstoffen mit Hilfe der Stoffwechselleistungen verschiedener Mikroorganismen Dicarbonsäuren präparativ zu erzeugen. So wird in der deutschen Patentschrift 21 40 133 vorgeschlagen, auf Alkane, primäre Alkanole oder Carbonsäuren eine Hefe der Gattung Candida und Pichia unter Fermentationsbedingungen einwirken zu lassen. Bei Einsatz von zum Beispiel einem speziellen Stamm von Candida lipolytica entstehen dabei Dicarbonsäuren deren C-Kette gegenüber dem Ausgangsmaterial nicht oder nicht wesentlich abgebaut ist und auch ansonsten keine Veränderungen wie Einführen neuer funktioneller Gruppen aufweist. Ähnliche Verfahren, bei denen spezielle andere Mikroorganismen eingesetzt werden, sind beschrieben in den US-Patenten 3 975 234 und 4 339 536; in der britischen Patentschrift 1 405 026 sowie in den deutschen Offenlegungsschriften 21 64 626, 28 53 847, 29 37 292 und 29 51 177.

Das US-Patent 4 474 882 hat ungesättigte Dicarbonsäuren zum Gegenstand. Diese werden gewonnen, indem ein Stamm der Spezies Candida tropicalis zur Umwandlung von ungesättigten Monocarbonsäuren mit 14 bis 22 C-Atomen eingesetzt wird. Die ungesättigten Dicarbonsäuren entsprechen in der Anzahl und Lage der Doppelbindung den Ausgangsmaterialien. Es werden also keine weiteren Doppelbindungen eingeführt.

Für den Chemiker sind ungesättigte Dicarbonsäuren sehr viel wertvollere Rohstoffe als gesättigte, da sie bisher nur in Einzelfällen im Labormaßstab zugänglich waren. Die Stoffe können aufgrund der Doppelbindung derivatisiert werden und so beispielsweise auch in gesättigte umgewandelt werden. Ungesättigte Dicarbonsäuren können wichtige Modifizierungsmittel für beispielsweise Polymere sein oder als Ausgangsstoffe für weitere Synthesen eingesetzt werden.

Aufgabe der Erfindung ist es daher, ein fermentatives Verfahren zu schaffen, daß zu Dicarbonsäuren mit einer höheren Anzahl an CC-Doppelbindungen führt. Gegenstand der Erfindung ist somit ein Verfahren zur Umwandlung von unverzweigten

- Alkanen mit 10 bis 24 C-Atomen, die gewünschtenfalls 1 bis 3 innenständige konjugierte oder isolierte Doppelbindungen und/oder eine endständige primäre OH-Gruppe aufweisen und/oder
- von den analogen Monocarbonsäuren mit gewünschtenfalls einer sekundären OH-Gruppe
- und/oder von den Estern der analogen Monocarbonsäuren mit Alkoholen der Funktionalität f = 1 bis 4, wobei die Alkohole eine Anzahl von C-Atomen im Bereich von bis zu 22 C-Atomen im Alkoholrest aufweisen unter üblichen Fermentationsbedingungen, gewünschtenfalls in Gegenwart von Hilfsstoffen in Dicarbonsäuren bzw. im Falle der Carbonsäureester in Dicarbonsäuremonoester mit gleicher Anzahl an C-Atomen, dadurch gekennzeichnet, daß in Gegenwart einer Blockmutante von Candida lipolytica mit der Hinterlegungsbezeichnung DSM 3581 und/oder deren zur Bildung ungesättigter Dicarbonsäuren befähigten Mutanten unter zumindest teilweiser Ausbildung von 1 bis 3 zusätzlichen CC-Doppelbindungen umgewandelt wird. Die erfindungsgemäß eingesetzten Blockmutanten von Candida lipolytica werden vorzugsweise von dem Wildstamm Candida lipolytica CBS 2075 abgeleitet. Der Wildstamm ist in der Lage, auf Fettsäuren und deren Derivaten zu wachsen und diese mit vergleichsweise hoher Geschwindigkeit abzubauen. Er ist daher als solcher nicht für das erfindungsgemäße Verfahren geeignet.

Der genannte Wildstamm wird seit 1978 auch unter der identischen Spezies-Bezeichnung Saccharomycopsis lipolytica CBS 2075 beim Centralbureau voor Schimmelcultures in Delft/Niederlande geführt.

Für das erfindungsgemäße Verfahren werden Mutanten des genannten oder vergleichbare Wildstämme eingesetzt, die nicht die Fähigkeit besitzen, die verfahrensgemäß eingesetzten Substrate unter Abbau der Kohlenstoffketten zu verändern. Entsprechende Mutantenstämme lassen sich durch Mutation und Selektion, insbesondere durch mehrfache Mutation und mehrfache Selektion gewinnen. Die Mutation des ausgewählten Wildstammes erfolgt in an sich bekannter Weise. Sie kann beispielsweise durch energiereiche Bestrahlung oder mit UV- oder Röntgenstrahlung erfolgen. Geeignet ist insbesondere aber auch die Behandlung der Zellen mit mutagenen Agenten. Geeignete mutagene Agentien sind beispielsweise Nitrosoguanidinverbindungen wie beispielsweise 1-Methyl-3-nitro-1-nitrosoguanidin oder Ethylmethansulfonat. Im einzelnen kann hier auf die allgemeinen Angaben des Standes der Technik verwiesen werden, siehe hierzu beispielsweise US-Patentschrift 4 029 549, Spalte 2, Zeilen 57 ff., mit den dort enthaltenen Verweisungen.

Zur Herstellung von Mutanten, die für das erfindungsgemäße Verfahren geeignet sind, werden die Bedingungen von Konzentration und Einwirkzeit des mutagenen Agens so gewählt, daß die Ausgangspopulation durch die Behandlung zu 10 bis 99,999 Prozent inaktiviert wird. Vorzugsweise wird eine Abtötungsrate von 80 bis 99,9 Prozent gewählt.

Zur nachfolgenden Auslese der erfindungsgemäß geeigneten Mutanten aus der großen Population der Mikroorganismen nach der Mutationsbehandlung werden Kulturbedingungen gewählt, unter denen die abgeänderten spezifischen Eigenschaften der entstandenen Mutantenstämme zum Selektionsvorteil werden oder erkennbar werden. Zur Selektion kann die nach der Mutation erhaltene Mikroorganismenpopulation beispielsweise auf einem Minimalmedium angebrütet werden, das als einzige Kohlenstoffquelle die im erfindungsgemäßen Verfahren eingesetzten Substrate enthält. Diejenigen mutierten Mikroorganismen, die aufgrund der Mutationsbehandlung ihre Fähigkeit verloren haben, auf der jetzt vorliegenden Kohlenstoffquelle zu wachsen, können sich beim Bebrüten des Trennmediums nicht vermehren. Sie wachsen also nicht. Ein anderer Teil der Mutantenpopulation, der entweder bei der Mutationsbehandlung nicht hinreichend geschädigt worden ist oder andere Defekte erlitten hat, ist befähigt, auf der Kohlenstoffquelle des Trennmediums zu wachsen. Bei der Bebrütung tritt also eine Vermehrung ein. Um eine Trennung zu erreichen, enthält das Trennmedium Stoffe, die bewirken, daß die wachsenden Stämme aufgrund ihres Wachstums oder während ihres Wachstums abgetötet werden, ohne daß dabei die nicht wachsenden Mutantenstämme geschädigt werden. Möglich ist eine solche Trennung beispielsweise durch Zusatz von Antibiotika.

Eine andere Möglichkeit der Mutantentrennung in diesem Stadium ist der Einbau von zellschädigenden, insbesondere reaktiven Komponenten in den auf dem Trennmedium wachsenden Anteil der Mutantenpopulation. Geeignet ist beispielsweise der Einbau von $P^{32}$ in die wachsenden Stämme. Möglich ist das beispielsweise durch Mitverwendung entsprechend radioaktiv markierter Salze der Phosphorsäure in der Nährlösung dieses Selektionsschritts. Für die Trennung mittels $P^{32}$ hat sich insbesondere die Mitverwendung von $NaH_2{}^{32}PO_4$ in der Nährlösung bewährt.

Aus den somit angereicherten Mutanten können dann zum Beispiel mittels der an sich bekannten Lederbergschen Stempelmethode die erfindungsgemäß angestrebten Mutantenstämme (Defektblockmutantenstämme) isoliert werden. Bezüglich der hier eingesetzten Verfahrensweise und zur Antibiotika-Anreicherungsmethode und zur Lederbergschen Stempelmethode wird beispielsweise verwiesen auf J. Amer. Chem. Soc. 70, 4267, (1948) Davis, B.D. (Antibiotika-Anreicherungsmethode), J. Bact. 63, 399 (1952) Lederberg, J., Lederberg, E. M. (Lederbergsche Stempelmethode).

Zur weiteren Selektion der Defektblockmutanten wird der Fachmann deren Fähigkeit zur Umwandlung der im erfindungsgemäßen Verfahren eingesetzten Substrate in die Carbonsäuren berücksichtigen, das heißt, es wird das Ergebnis der Fermentation hinsichtlich Dicarbonsäuregehalt und hinsichtlich der in den Dicarbonsäuren vorhandenen neu eingeführten. Doppelbindungen berücksichtigt. Dabei können besonders günstige Ergebnisse erzielt werden, wenn Mutations- und Selektionsschritt mehrfach durchgeführt werden.

Eine für das erfindungsgemäße Verfahren besonders geeignete Defektblockmutante ist der Mikroorganismus Candida lipolytica DSM 3581 (Hinterlegungsbezeichnung). Weiter geeignet sind die Mutanten dieses Stammes soweit sie zur Bildung ungesättigter Dicarbonsäuren befähigt sind.

Als Ausgangsmaterialien können für das erfindungsgemäße Verfahren verzweigte Alkane mit 10 bis 24 C-Atomen, die gewünschtenfalls 1 bis 3 innenständige konjugierte oder isolierte Doppelbindungen aufweisen, eingesetzt werden. Die Alkane können als definierte chemische Verbindungen oder als Mischung eingesetzt werden. So können beispielsweise Alkangemische eingesetzt werden wie sie aus Mineralöl erhalten werden. Weiterhin eingesetzt werden können auch Oligomerisierungsprodukte des Ethylen.

Nach einer weiteren bevorzugten Ausführungsform der Erfindung können primäre Alkohole mit 10 bis 22 C-Atomen eingesetzt werden. Auch die Alkohole können als definierte Verbindung oder als Mischung eingesetzt werden. Zwei Gruppen von primären Alkoholen sind von besonderer Bedeutung. Zum einen die sogenannten Fettalkohole, das sind Mischungen von gesättigten und ungesättigten Alkoholen, die in ihrer Kettenlängenverteilung und Anzahl und Lage der Doppelbindungen den Fettsäureschnitten entsprechen, aus denen sie hergestellt worden sind. Fettalkohole enthalten geradzahlige Alkohole mit 1 bis 3 Doppelbindungen. Besonders wichtige Bestandteile sind im Hinblick auf das erfindungsgemäße Verfahren Fettalkohole der Kettenlänge $C_{16}$, $C_{18}$ und $C_{22}$, gesättigt oder mit einer Doppelbindung. Eine weitere Gruppe von primären Alkoholen sind die durch Oligomerisierung von Ethylen zugänglichen Alkohole (Ziegler-Alkohole). Dabei handelt es sich um primäre, unverzweigte, gesättigte Alkohole mit ebenfalls einer geraden Anzahl von Kohlenstoffatomen. Auch Alkohole mit ungerader Anzahl von C-Atomen verschiedener Provenienz können eingesetzt werden.

Nach einer weiteren Ausführungsform der Erfindung können auch Monocarbonsäuren eingesetzt werden. Hier sind unverzweigte Monocarbonsäuren mit endständiger Carboxylgruppe geeignet, das heißt Verbindungen, die in der Anzahl der C-Atome und Zahl und Lage der Doppelbindungen den vorgenannten Alkoholen entsprechen. Eine wichtige Gruppe von Monocarbonsäuren sind Fettsäuren, wie sie den natürlich vorkommenden Fettsäuretriglyceriden (Fette) zu Grunde liegen. Eingesetzt werden können Fettsäuregemische mit unterschiedlicher Zusammensetzung, das heißt sowohl destillierte, vorgereinigte Produkte wie auch die natürlich vorkommenden Mischungen. Auf diesem Gebiet stehen dem Fachmann Produkte zur Verfügung, die einen hohen oder einen niederen Grad an Unsättigung aufweisen. Beispiel für Fettsäuregemische sind die Fettsäuren, die aus Cocosöl, Palmkernöl, Palmöl, Erdnußöl, Baumwollsaatöl, Sojabohnenöl, Sonnenblumenöl, Leinöl, Rüböl, tierischen Fetten oder seetierischen Fetten herstellbar sind. Ein weiterer wichtiger Rohstoff ist die Fettsäure, die aus Ricinusöl gewonnen werden kann. Diese Fettsäure besteht zum großen Teil aus 12-Hydroxyoctadecensäure. Darüber hinaus können weitere Monocarbonsäuren mit einer sekundären Hydroxylgruppe eingesetzt werden, beispielsweise die 9- oder 10-Hydroxystearinsäure.

Eine weitere Gruppe von Monocarbonsäuren, die im erfindungsgemäßen Verfahren als Ausgangsstoffe eingesetzt werden können, sind die zu den vorgenannten durch Oligomerisierung von Ethylen zugänglichen Alkoholen homologen Carbonsäuren. Dabei handelt es sich größtenteils um gesättigte Carbonsäuren. Darüber hinaus können auch Monocarbonsäuren mit einer ungeraden Anzahl an C-Atomen verschiedener Provenienz eingesetzt werden.

Nach einer weiteren Variante des erfindungsgemäßen Verfahrens können auch Ester der vorgenannten Monocarbonsäuren eingesetzt werden. Geeignet sind hier Ester mit Alkoholen der Funktionalität 1 bis 4 und bis zu 22 C-Atomen im Alkoholrest. Eine besonders bevorzugte Klasse von Estern sind die Ester der genannten Carbonsäure mit $C_1$ bis $C_4$-Alkoholen. Unter $C_1$ bis $C_4$-Alkoholen sind hier zum einen die primären Alkohole mit 1 bis 4 C-Atomen, die sekundären Alkohole mit 3 oder 4 C-Atomen, Diole mit 2 bis 4 C-Atomen, Triole mit 3 bis 4 C-Atomen oder Tetraole mit 4 C-Atomen geeignet.

Nach einer besonders bevorzugten Ausführungsform der Erfindung werden natürlich vorkommende Fettsäuretriglyceride als Ausgangsmaterial eingesetzt. Hier eignen sich die vorstehend bei den Fettsäuren diskutierten öligen oder talgartig festen Triglyceride. Bevorzugt sind flüssige Triglyceride.

Eine weitere Variante des erfindungsgemäßen Verfahrens betrifft den Einsatz von Estern langkettiger Carbonsäuren mit langkettigen primären Alkoholen. So können insbesondere die Ester von Fettsäuren mit Fettalkoholen eingesetzt werden.

Das zur Durchführung des erfindungsgemäßen Verfahrens eingesetzte Fermentationsmedium entspricht den für die Anzucht von Candida-Arten, insbesondere von Mikroorganismen der Spezies Candida lipolytica bekannten und beschriebenen Medien. Geeignete Fermentationsmedien enthalten die üblichen Spurenelemente, eine Stickstoffquelle und eine zusätzliche, dem zu transformierenden Substrat nicht identische Kohlenstoffquelle. Unter Spurenelementen sind hier Salze der Kationen, Ammonium, Kalium, Natrium, Magnesium, Calcium und Mangan mit den Anionen Phosphat, Sulfat, Chlorid zu verstehen. Als Stickstoffquelle können neben anorganischen Verbindungen wie zum Beispiel $(NH_4)_2SO_4$ auch Pepton oder Hefeextrakt eingesetzt werden.

Die Fermentationslösung enthält weiterhin als Kohlenstoffquelle ein Cosubstrat. Als Cosubstrat kann beispielsweise Natriumacetat eingesetzt werden. Weiterhin ist es möglich, daß als Cosubstrat übliche Zucker wie Glucose, Fructose, Maltose, Trehalose und dergleichen eingesetzt werden. Ein besonders bevorzugtes Cosubstrat ist Glycerin.

Im Hinblick auf die Verfahrensführung hat sich gezeigt, daß die Ausbeute an Dicarbonsäuren sich steigern läßt, wenn das Cosubstrat, also die Kohlenstoffquelle, nicht am Anfang der Fermentation sondern kontinuierlich in ausreichenden Mengen während der Umsetzung zugegeben werden.

Nach einer weiteren Ausführungsform des erfindungsgemäßen Verfahrens ist es bevorzugt, den zur Umwandlung vorgesehenen Ausgangsverbindungen zur besseren Verteilung im Fermentationsmedium Emulgatoren zuzufügen. So werden günstige Ergebnisse erzielt, wenn insgesamt 0,1 bis 3 Gew.-%, bezogen auf Fermentationsmedium, an Emulgatoren vorhanden sind. Geeignet sind hier physiologisch verträgliche Emulgatoren und insbesondere physiologisch verträgliche nichtionische Emulgatoren. So können beispielsweise Zuckerester oder auch Sorbitanester, ethoxylierte Zuckerester, ethoxylierte Sorbitanester oder auch Alkylglycoside eingesetzt werden.

Nach dem erfindungsgemäßen Verfahren werden die Ausgangsverbindungen in Dicarbonsäuremischungen überführt, die zumindest teilweise ungesättigte Dicarbonsäuren enthalten, welche ein oder zwei Doppelbindungen mehr als die Ausgangssubstrate enthalten. So kann molmäßig der Anteil an Dicarbonsäuren, die eine zusätzliche Doppelbindung aufweisen, in den Reaktionsprodukten mehr als die Hälfte betragen. Der Anteil an Dicarbonsäuren, die zwei zusätzliche Doppelbindungen enthalten, kann um 30 Prozent betragen. Die nach dem erfindungsgemäßen Verfahren hergestellten Dicarbonsäuregemische können darüber hinaus auch noch Anteile an gesättigten Carbonsäuren aufweisen.

Aufgrund analytischer Befunde wird vermutet, daß die neu eingeführten CC-Doppelbindungen in den Dicarbonsäuren sich in Dreistellung zu der oder zu den Carboxylgruppen befinden. Insbesondere können nach dem erfindungsgemäßen Verfahren aus Palmitinsäure bzw. Ölsäure, bzw. Ricinolsäure, die in der Anzahl an C-Atomen analogen Dicarbonsäuren mit zusätzlich einer bzw. zwei Doppelbindungen am Kohlenstoffatom 3 bzw. am Kohlenstoffatom N-3 (wobei N die Gesamtanzahl der Kohlenstoffatome in diesen Verbindungen bezeichnet) hergestellt werden. Die neuen ungesättigten Dicarbonsäuren können aus den bei der Fermentation entstandenen Gemischen nach konventionellen Trennmethoden isoliert werden. So können die Gemische nach Extraktion aus dem Fermentationsmedium oder nach Adsorption an Ionenaustauschern und Elution mit geeigneten Lösungsmitteln als solche oder in derivatisierter Form mit chromatographischen Methoden wie HPLC aufgetrennt werden.

Für viele Anwendungen jedoch sind die bei der Fermentation hergestellten ungesättigten Dicarbonsäuregemische direkt einsetzbar. So können die Gemische beispielsweise direkt zur Modifizierung von Polymeren, insbesondere Harzen eingesetzt werden.

## Beispiele

Eine Defektblockmutante eines Mikroorganismus der Spezies Candida lipolytica (DSM 3581 Hinterlegungsbezeichnung) wurde zunächst in einer Vorkultur 36 Stunden bei 30 °C aerob in 100 ml Würzebouillon angezüchtet.

10 ml der Vorkultur wurden in 100 ml Medium folgender Zusammensetzung eingeimpft (Angaben in Gew.-%):

3.00 % Glycerin
1.00 % Glukose,
0.30 % Hefeextrakt,
0.20 % $(NH_4)_2SO_4$,
0.05 % $MgSO_4$ $7H_2O$,
0.01 % $CaCl_2$ $2H_2O$,
0.01 % $MnSO_4$ $4H_2O$,
0.10 % $K_2HPO_4$,
0.05 % $NaH_2PO_4$, pH 6.2.

Die Kultur wurde bei 30 °C inkubiert. Nach 30 Stunden Kulturdauer erfolgte die Zugabe von 1 % Kohlenwasserstoff oder Monocarbonsäure als Substrat und nach weiteren 6 Stunden nochmals 9 % Substrat plus 1.0 % Glycerin als Cosubstrat. Der pH-Wert der Kulturlösung wurde zum Zeitpunkt der zweiten Substratzugabe auf pH 7.2 hochgestellt und dort gehalten.

Nach 96 bis 120 Stunden Kulturdauer wurden Proben gezogen, die Proben mit aliquoten Mengen Ether oder Acetessigester extrahiert und per DC sowie GC analysiert.

### Beispiel 1

Bei Einsatz von 30 g/l Palmitinsäure wurden 6 g/l eines Dicarbonsäuregemisches erhalten bestehend aus
ca. 60 % $\Delta$-3-$C_{16}$-Dicarbonsäure,
ca. 30 % $\Delta$-3,13-$C_{16}$-Dicarbonsäure und
ca. 10 % $C_{16}$-Dicarbonsäure.

### Beispiel 2

Bei Einsatz von 30 g/l Methylpalmitat wurden 7 g/l eines Dicarbonsäuregemisches in etwa gleicher Zusammensetzung wie in Beispiel 1 nachgewiesen.

### Beispiel 3

Bei Einsatz von 30 g/l Palmöl wurden etwa 9 g/l eines Dicarbonsäuregemisches erhalten, bestehend aus gesättigten sowie einfach, zweifach und dreifach ungesättigten Dicarbonsäuren.

### Beispiel 4

Bei Einsatz von 30 g/l Ricinusöl wurden etwa 4 g/l eines Dicarbonsäuregemisches erhalten, überwiegend bestehend aus
$\Delta$-3,9,15-12-Hydroxy-$C_{18}$-Dicarbonsäure,
$\Delta$-3,9-12-Hydroxy-$C_{18}$-Dicarbonsäure und
$\Delta$ 9-12-Hydroxy-$C_{18}$-Dicarbonsäure.

### Beispiel 5

Aufarbeitung: Nach Erreichen des Ausbeutemaximums in der Kultur wurde die Kulturlösung auf 80 °C erhitzt, der pH-Wert mit 12 n KOH auf 11,0 hochgestellt, die Zellen durch Filtration oder Zentrifugation abgetrennt und die Zellmasse mit verdünntem Alkali nachgewaschen. Filtrat und Waschwasser wurden vereinigt und die Dicarbonsäuren durch Absenken des pH-Wertes auf 4,0 mit konzentrierter $H_2SO_4$ ausgefällt. Zur optimalen Auskristallisierung wurde das Präzipitat 5 Stunden unter Umrühren auf 80 °C erhitzt, anschließend abfiltriert, mit verdünnter Säure und Wasser nachgewaschen und getrocknet.

## Patentansprüche

1. Verfahren zur Umwandlung von unverzweigten

- Alkanen mit 10 bis 24 C-Atomen, die gewünschtenfalls 1 bis 3 innenständige konjugierte oder isolierte Doppelbindungen und/oder eine endständige primäre OH-Gruppe aufweisen und/oder
- von den analogen Monocarbonsäuren mit gewünschtenfalls einer sekundären OH-Gruppe
- und/oder von den Estern der analogen Monocarbonsäuren mit Alkoholen der Funktionalität f = 1 bis 4, wobei die Alkohole eine Anzahl von C-Atomen im Bereich von bis zu 22 C-Atomen im Alkoholrest aufweisen

unter üblichen Fermentationsbedingungen, gewünschtenfalls in Gegenwart von Hilfsstoffen, in Dicarbonsäuren bzw. im Falle der Carbonsäureester in Dicarbonsäuremonoester mit gleicher Anzahl an C-Atomen, dadurch gekennzeichnet, daß in Gegenwart einer Blockmutante von Candida lipolytica mit der Hinterlegungsbezeichnung DSM 3581 und/oder deren zur Bildung ungesättigter Dicarbonsäuren befähigten Mutanten unter zumindest teilweiser Ausbildung von 1 bis 3 zusätzlichen CC-Doppelbindungen umgewandelt wird.

2. Verfahren nach Anspruch 1, dadurch gekennzeichnet, daß als Substrat Mischungen von unverzweigten, primären Alkoholen und/oder Monocarbonsäuren eingesetzt werden, die 12 bis 22 C-Atome und bis zu 3 CC-Doppelbindungen aufweisen, insbesondere Fettsäuren und/oder Fettalkohole.

3. Verfahren nach den Ansprüchen 1 bis 2, dadurch gekennzeichnet, daß als Substrat Ester von Monocarbonsäuren mit Alkoholen der Kettenlänge $C_1$ bis $C_4$, insbesondere Monoalkoholen, Thiolen oder Triolen eingesetzt werden.

4. Verfahren nach den Ansprüchen 1 bis 3, dadurch gekennzeichnet, daß als Substrat Fettsäuretriglyceride, insbesondere unter Fermentationsbedingungen flüssige Fettsäuretriglyceride eingesetzt werden.

5. Verfahren nach den Ansprüchen 1 bis 4, dadurch gekennzeichnet, daß als Substrat die Ester aus linearen Carbonsäuren mit 10 bis 24 C-Atomen und linearen primären Alkoholen mit 5 bis 22 C-Atomen, insbesondere Ester von Fettsäuren mit Fettalkoholen eingesetzt werden.

6. Verfahren nach den Ansprüchen 1 bis 5, dadurch gekennzeichnet, daß als Hilfsstoffe Co-Substrate eingesetzt werden, vorzugsweise physiologisch verträgliche mehrfunktionelle Alkohole wie Zucker, Zuckeralkohole oder Glycerin.

7. Verfahren nach den Ansprüchen 1 bis 6, dadurch gekennzeichnet, daß als Hilfsstoffe physiologisch verträgliche Emulgatoren eingesetzt werden.

8. Verfahren nach den Ansprüchen 1 bis 7, dadurch gekennzeichnet, daß das Co-Substrat während der Fermentationsdauer portionsweise zugesetzt wird.

9. Mikroorganismus Candida lipolytica DSM 3581 und dessen zur Durchführung des Verfahrens nach Anspruch 1 befähigte Blockmutanten.

**Claims**

1. A process for the conversion of unbranched
   - $C_{10-24}$ alkanes which, if desired, may contain from 1 to 3 internal conjugated or isolated double bonds and/or a terminal primary OH-group and/or
   - the analogous monocarboxylic acids, if desired containing a secondary OH-group
   - and/or esters of the analogous monocarboxylic acids with alcohols having a functionality f of 1 to 4, the alcohols containing up to 22 carbon atoms in the alcohol radical,

   under standard fermentation conditions, if desired in the presence of auxiliaries, into dicarboxylic acids or - in the case of carboxylic acid esters - into dicarboxylic acid monoesters containing the same number of carbon atoms, characterized in that the conversion is carried out in the presence of a block mutant of Candida lipolytica deposited under the name DSM 3581 and/or mutants thereof capable of forming unsaturated dicarboxylic acids with at least partial formation of 1 to 3 additional CC-double bonds.

2. A process as claimed in claim 1, characterized in that mixtures of unbranched primary alcohols and/or monocarboxylic acids containing from 12 to 22 carbon atoms and up to 3 CC-double bonds, more especially fatty acids and/or fatty alcohols, are used as the substrate.

3. A process as claimed in claims 1 and 2, characterized in that esters of monocarboxylic acids with $C_{1-4}$ alcohols, particularly monoalcohols, thiols or triols are used as the substrate.

4. A process as claimed in claims 1 to 3, characterized in that fatty acid triglycerides, particularly fatty acid triglycerides which are liquid under fermentation conditions, are used as the substrate.

5. A process as claimed in claims 1 to 4, characterized in that esters of linear $C_{10-24}$ carboxylic acids and linear primary $C_{5-22}$ alcohols, particularly esters of fatty acids with fatty alcohols, are used as the substrate.

6. A process as claimed in claims 1 to 5, characterized in that co-substrates, preferably physiologically safe polyfunctional alcohols, such as sugars, sugar alcohols or glycerol, are used as the auxiliaries.

7. A process as claimed in claims 1 to 6, characterized in that physiologically safe emulsifiers are used as the auxiliaries.

8. A process as claimed in claims 1 to 7, characterized in that the co-substrate is added in portions during the fermentation time.

9. Microorganism Candida lipolytica DSM 3581 and block mutants thereof suitable for carrying out the process claimed in claim 1.

**Revendications**

1. Procédé de transformation
   - d'alcanes linéaires comportant 10 à 24 atomes de C, qui présentent éventuellement 1 à 3 doubles liaisons conjuguées en position interne ou isolées et/ou un groupement OH primaire terminal et/ou
   - des acides monocarboxyliques analogues comportant éventuellement un groupe OH secondaire
   - et/ou des esters des acides monocarboxyliques analogues avec des alcools de fonctionnalité f = 1 à 4, lesdits alcools comportant un nombre d'atomes de C allant jusqu'à 22 atomes de C dans le radical alcool,
   
   dans les conditions de fermentation habituelles, le cas échéant en présence d'adjuvants, en acides dicarboxyliques ou, dans le cas des esters d'acides carboxyliques, en monoesters d'acides dicarboxyliques comportant un nombre d'atomes de C identique, caractérisé en ce que ladite transformation a lieu en présence d'un mutant en bloc de Candida lipolytica portant la dénomination déposée DSM 3581 et/ou de ses mutants aptes à la production d'acides dicarboxyliques insaturés, avec formation au moins partielle de 1 à 3 doubles liaisons CC supplémentaires.

2. Procédé selon la revendication 1, caractérisé en ce l'on met en oeuvre comme substrat des mélanges d'alcools primaires linéaires et/ou d'acides monocarboxyliques, qui possèdent 12 à 22 atomes de C et jusqu'à 3 doubles liaisons CC, en particulier des acides gras et/ou des alcools gras.

3. Procédé selon les revendications 1 à 2, caractérisé en que l'on met en oeuvre comme substrat des esters d'acides monocarboxyliques avec des alcools dont la longueur de chaine est comprise entre $C_1$ et $C_4$, en particulier des mono-alcools, des thiols (diols) ou des triols.

4. Procédé selon les revendications 1 à 3, caractérisé en ce que l'on met en oeuvre comme substrat des triglycérides d'acides gras, en particulier des triglycérides d'acides gras liquide dans des conditions de fermentation.

5. Procédé selon les revendications 1 à 4, caractérisé en ce que l'on met en oeuvre comme substrat les esters d'acides carboxyliques linéaires comportant 10 à 24 atomes de C avec des alcools primaires comprenant 5 à 22 atomes de C, en particulier des esters d'acides gras avec des alcools gras.

6. Procédé selon les revendications 1 à 5, caractérisé en ce que l'on met en oeuvre comme adjuvants, des co-substrats, en particulier des alcools polyfonctionnels physiologiquement tolérables, tels que sucres, alcools de sucre ou glycérine.

7. Procédé selon les revendications 1 à 6, caractérisé en ce que l'on met en oeuvre comme adjuvants, des émulsifiants physiologiquement tolérables.

8. Procédé selon les revendications 1 à 7, caractérisé en ce que l'on met en oeuvre le co-substrat par portions pendant le déroulement de la fermentation.

9. Microorganisme Candida lipolytica DSM 3581 et ses mutants en bloc aptes à la réalisation du procédé selon la revendication 1.